# EUROPÄISCHE PATENTSCHRIFT

(11) **EP 2 387 554 B1**
(45) Veröffentlichungstag und Bekanntmachung des Hinweises auf die Patenterteilung: **14.01.2015**
(21) Anmeldenummer: 09799279.6
(22) Anmeldetag: 21.12.2009
(51) Int. Cl.: C07C 45/50, C07C 47/02

(54) **VERFAHREN ZUR HERSTELLUNG VON ALDEHYDEN**
METHOD FOR PRODUCING ALDEHYDES
PROCÉDÉ DE PRODUCTION D'ALDÉHYDES

(30) Priorität: 15.01.2009 DE 102009004655
(43) Veröffentlichungstag der Anmeldung: 23.11.2011
(73) Patentinhaber: OXEA GmbH, 46147 Oberhausen (DE)
(72) Erfinder: GREB, Wolfgang, 46535 Dinslaken (DE); ARNOLD, Jörg, 46535 Dinslaken (DE); KONIETZNY, Gregor, 46147 Oberhausen (DE); ZGORZELSKI, Wolfgang, 46149 Oberhausen (DE); SCHALAPSKI, Kurt, 46147 Oberhausen (DE)
(86) Internationale Anmeldenummer: PCT/EP2009/009197
(87) Internationale Veröffentlichungsnummer: WO 2010/081526

(56) Entgegenhaltungen:
- EP-A1- 0 805 138
- WO-A1-2006/108698
- US-A1- 2004 059 172
- DATABASE WPI Week 200320 Thomson Scientific, London, GB; AN 2003-204129 XP002586422 & JP 2002 331243 A (KURARAY CO LTD) 19. November 2002 (2002-11-19)

## Beschreibung

Die Erfindung betrifft ein verbessertes Verfahren zur Hydroformylierung olefinisch ungesättigter Verbindungen in Gegenwart einer wässrigen, wasserlösliche Rhodium-Komplexverbindungen enthaltenden Katalysatorlösung und insbesondere die Nutzung der mit dem Abgas aus der Hydroformylierungszone entweichenden, nicht umgesetzten Olefine.

Es ist bekannt, dass Verbindungen, die olefinische Doppelbindungen enthalten, mit Kohlenmonoxid und Wasserstoff zu Aldehyden umsetzbar sind (Oxosynthese). Der Prozess ist nicht auf den Einsatz olefinischer Kohlenwasserstoffe begrenzt, sondern erstreckt sich auch auf Ausgangsstoffe, die außer der Doppelbindung noch funktionelle Gruppen aufweisen, vorwiegend solche, die unter den Reaktionsbedingungen unverändert bleiben.

Die klassische Oxosynthese arbeitet mit Kobalt als Katalysator. Seine Wirksamkeit beruht auf der Bildung von Kobaltcarbonylverbindungen unter der Einwirkung von Wasserstoff und Kohlenmonoxid bei Drücken oberhalb 20 MPa und Temperaturen von etwa 120°C und mehr auf metallisches Kobalt oder Kobaltverbindungen.

Im Laufe der Weiterentwicklung der Oxosynthese wurde Kobalt zunehmend durch Rhodium als Katalysatormetall ersetzt. Rhodium wird als Komplexverbindung eingesetzt, das neben Kohlenmonoxid vorzugsweise Phosphine als Liganden enthält. Rhodium als Metall erlaubt es, bei niedrigen Drücken zu arbeiten, überdies erzielt man höhere Ausbeuten und bevorzugt werden die für die Weiterverarbeitung wertvolleren unverzweigten Produkte gebildet, wenn man von geradkettigen endständigen Olefinen ausgeht.

Eine weitere technische Entwicklung der Oxosynthese bedeutete der Übergang vom homogenen zum zweiphasigen im Reaktionsmedium, d.h. im Einsatzmaterial und im Rohprodukt gelösten Katalysatoren zu wässrigen Katalysatorlösungen, die als eigene Phase getrennt von Einsatzstoffen und Reaktionsprodukten vorliegen. Diese Variante der Oxosynthese ist z.B. aus DE-B-26 27 354 bekannt. Ihr besonderer Vorteil ist die leichte Trennung von Reaktionsprodukt und Katalysator, die schonend, ohne Anwendung thermischer Verfahrensschritte erfolgt und daher Verluste vermeidet, die durch Folgereaktionen der entstandenen Aldehyde eintreten. Weiterhin erzielt man sehr hohe Ausbeuten und bei der Verwendung unverzweigter endständständiger Olefine erhält man ganz überwiegend n-Aldehyde. Diese Verfahrensvariante wird auch als heterogenes oder zweiphasiges Verfahren bezeichnet.

Aus Gründen der Prozessökonomie, insbesondere um große Reaktoren oder lange Reaktionszeiten zu vermeiden, führt man die Umsetzung nicht bis zum vollständigen Verbrauch der olefinisch ungesättigten Verbindungen, sondern begnügt sich häufig mit der Umwandlung von lediglich 60 bis 95 % des Ausgangsmaterials zur gewünschten Endverbindung. Im Abgas, das die Hydroformylierungszone verlässt, befinden sich daher neben überschüssigem Kohlenmonoxid und Wasserstoff nicht umgesetztes olefinisches Einsatzmaterial, das nach unterschiedlich ausgestalteten Verfahren in Wertstoffe umgewandelt werden kann.

Gemäß EP-B1-0 111 257 setzt man Abgas aus einer ersten Hydroformylierungsstufe, in der Olefin mit Kohlenmonoxid und Wasserstoff nach dem zweiphasigen Hydroformylierungsverfahren umgesetzt werden, in einer zweiten Stufe nach dem klassischen Oxoverfahren bei hohem Druck in Gegenwart von Kobaltkatalysatoren um.

Eine Weiterentwicklung dieses Prozesses ist aus EP-A1-0 805 138 bekannt, in dem das Abgas der ersten, nach heterogener Fahrweise durchgeführten Hydroformylierungsreaktion in einer zweiten Stufe in einem homogenen Reaktionssystem in Gegenwart von Komplexverbindungen des Rhodiums mit organischen Phosphor(III)-Verbindungen als Katalysatoren umgesetzt wird.

Dieses Verfahren ermöglicht, dass der größte Teil der im Abgas enthaltenen, in der ersten Stufe nicht umgesetzten olefinischen Verbindung hydroformyliert wird. Die Vorteile dieses Prozesses waren nicht vorherzusehen. Zum einen liegen die olefinisch ungesättigten Verbindungen im Abgas in beträchtlicher Verdünnung vor, etwa mit einem Gehalt von 20 bis 50 Gew.-%. Derartige Konzentrationen stehen bei dieser Reaktionsart einer weitgehenden Umsetzung der ungesättigten Ausgangsverbindung entgegen. Ebenfalls werden mit dem Abgas aus der ersten Reaktionsstufe Verunreinigungen ausgetragen, beispielsweise schwefelhaltige Abbauprodukte des Katalysatorsystems wie Mercaptane, sowie Wasser. Sowohl die in dem organischen Medium der zweiten, homogen durchgeführten Reaktionsstufe gut löslichen Mercaptane als auch Wasser können den homogen gelösten Rhodiumkomplexkatalysator schädigen und zur Bildung von katalytisch inaktiven Verbindungen führen. Trotz dieser zunächst erwarteten Nachteile kann der aus EP 0 805 138 A1 bekannte Prozess mit hoher Effizienz durchgeführt werden.

Das Abstract von JP 2002 331243 A behandelt die Rückgewinnung eines Rhodiumkatalysators aus dem Gemisch einer Hydroformylierungsreaktion. Das im Polyethylenglykoldimethylether als Lösungsmittel unter Katalyse mit einem Rhodium- monosulfoniertem Triphenylphosphin-Komplex erhaltene Reaktionsgemisch wird mit Wasser versetzt. Durch intensives Rühren wird eine Emulsion gebildet, die anschließend mit Hilfe eines Koalisierfilters in die wässrige und organische Phase getrennt wird.

US 2004/059172 A1 betrifft die Alkylierung von Olefinen mit Isoalkanen in Gegenwart einer wässrigen Schwefelsäure als Katalysator, wobei das Reaktionsprodukt zur Entfernung der wässrigen Schwefelsäure über eine Entwässerungseinrichtung mit einem Koalisierfilter geleitet wird.

WO 2006/108698 A1 offenbart den Einsatz eines Koalisierfilters im Rahmen der Wäsche eines Olefingemisches. Das gewaschene und vom Wasser befreite Olefingemisch wird anschließend oligomerisiert und dann hydroformyliert. Die aus den Hydroformylierungsprodukten gewonnenen Alkohole werden zur Wasserentfernung mit einem Koalisierfilter in Kontakt gebracht.

Dennoch ist nach dem bekannten Verfahren nicht immer eine optimale Prozessführung möglich. So können bei schwankenden Betriebszuständen, die im industriellen Betrieb gelegentlich auftreten, geringe Mengen der wässrigen Katalysatorlösung aus der ersten Hydroformylierungsstufe mit dem Abgas ausgetragen werden und in die zweite homogen durchgeführte Hydroformylierungsstufe gelangen. Dabei können sich Wasser und die zuvor genannten schwefelhaltigen Verbindungen in der zweiten, homogenen Hydroformylierungsstufe in einer solchen Menge anreichern, dass das homogene Katalysatorsystem in erheblichem Maße geschädigt wird und die Effizienz des Hydroformylierungsprozesses leidet. Ebenfalls komplexiert ausgetragener, wasserlöslicher Ligand aus der ersten Hydroformylierungsstufe das in der zweiten Stufe anwesende Rhodium zu Komplexverbindungen, die im homogenen Reaktionsmedium katalytisch nicht mehr aktiv sind. Auch bei längerem Betrieb unter optimalen Betriebszuständen können sich Wasser und schwefelhaltige Verbindungen in dem organischen Medium der zweiten Hydroformylierungsstufe anreichern und den Hydroformylierungskatalysator schädigen. Auch wird das in die zweite Hydroformylierungsstufe eingetragene Wasser teilweise mit den gebildeten Aldehyden aus dem Hydroformylierungsprozess ausgeschleust. Es muss dann bei der folgenden destillativen Aufarbeitung der Aldehyde oder bei der destillativen Aufarbeitung der nach Hydrierung erhaltenen Alkohole unter Energieaufwand entfernt werden.

Es bestand daher die Aufgabe, ein Hydroformylierungsverfahren bereitzustellen, dass es auf einfache Weise erlaubt, die Wirtschaftlichkeit eines Hydroformylierungsprozesses zu verbessern, bei dem olefinisch ungesättigte Verbindungen, die im Abgas einer mit wässriger Katalysatorlösung durchgeführten Hydroformylierungsreaktion enthalten sind, zu Carbonylverbindungen umgesetzt werden, und die Wirtschaftlichkeit der nachfolgenden Aufarbeitungsprozesse für die Carbonylverbindungen oder für die daraus hergestellten Folgeprodukte zu verbessern.

Die Erfindung besteht daher in einem Verfahren zur Hydroformylierung olefinisch ungesättigter Verbindungen, wobei die Reaktion der ersten Reaktionsstufe in einem heterogen Reaktionssystem unter Verwendung einer wässrigen Lösung, wasserlösliche organische Phosphor(III)-Verbindungen in komplexer Bindung enthaltender Rhodiumverbindungen als Katalysatoren bei Drücken von 0,4 bis 10 MPa erfolgt und Abgas gebildet wird, und wobei das Abgas der ersten Reaktionsstufe einer zweiten Reaktionsstufe zugeführt wird, in der im Abgas noch vorhandene Restmengen der olefinisch ungesättigten Verbindungen in einem homogenen Reaktionssystem in Gegenwart von Komplexverbindungen des Rhodiums mit organischen Phosphor(III)-Verbindungen als Katalysatoren bei Drücken von 15 bis 40 MPa umgesetzt werden. Es ist dadurch gekennzeichnet, dass man das Abgas der ersten Reaktionsstufe kühlt und ein flüssiges Abgaskondensat bildet, das man durch einen Koalisierfilter leitet, um Wasser und darin enthaltene Inhaltsstoffe zu entfernen, und anschließend der zweiten Reaktionsstufe zuführt.

Durch die erfindungsgemäße Maßnahme, das Abgas der ersten Reaktionsstufe zunächst unter Bildung eines flüssigen Abgaskondensats zu kühlen und anschließend durch einen Koalisierfilter zu leiten, können vielfältige Vorteile erzielt werden. Die mit dem Abgas aus der ersten Hydroformylierungsstufe mitgerissenen Wasserspuren bilden bei dem Abkühlungsschritt kleinste, in der organischen Phase dispergierte Tropfen, die bei Kontakt mit der Oberfläche des Koalisierfilters einen benetzenden Film bilden. Mit dem treibenden Strom des flüssigen Abgaskondensats wandert dieser Film durch das Filter bis zum Austritt, wo sich dann periodisch große Wassertropfen abtrennen und alleine aufgrund ihrer Schwerkraft abscheiden und in einem zusätzlichen Abscheider aufgefangen werden. Auf diese Weise kann das im Abgaskondensat der ersten Hydroformylierungsstufe enthaltene Wasser auf mechanischem Wege sehr einfach aus dem Prozess ausgeschleust werden. Auch im Wasser gelöste Substanzen, beispielsweise mitgerissene organische Phosphor(III)-Verbindungen, die in der ersten Hydroformylierungsstufe als Liganden verwendet werden, oder Kohlendioxid und daraus gebildete Kohlensäure können so entfernt werden. Es hat sich nämlich gezeigt, dass im Abgaskondensat der ersten Hydroformylierungsstufe enthaltene Wasserreste, in denen wasserlösliche organische Phosphor(III)-Verbindungen enthalten sind, in der zweiten, homogen durchgeführten Hydroformylierungsreaktion schädlich für den homogen gelösten Rhodiumkatalysator sind.

Durch das Zwischenschalten eines Koalisierfilters zwischen der ersten und zweiten Hydroformylierungsstufe und durch das auf diese Weise vorgenommene Ausschleusen des mitgerissenen Wassers, lassen sich auch in den nachgeschalteten Reinigungsprozessen der gebildeten Aldehyde und deren Folgeprodukte weitere Vorteile erzielen. Da bereits Wasser vorab auf mechanischem Wege aus dem zweistufigen Hydroformylierungsprozess ausgeschleust wird, kann die anschließende destillative Aufarbeitung zur Wasserabtrennung unter geringerem Energieaufwand betrieben zu werden. Auch gestaltet sich die Weiterverarbeitung der Aldehyde mit einem geringeren Wassergehalt einfacher.

Bei den im erfindungsgemäßen Verfahren eingesetzten Koalisierfiltern handelt es sich um flüssig/flüssig Phasentrenner zur Feinsttropfenabscheidung, bei denen die physikalische Wirkung der Koaleszenz in speziell aufgebauten, zylindrischen Faserbett-Elementen ausgenutzt wird. Derartige Koalisierfilter sind aus dem Stand der Technik bekannt, beispielsweise aus Chemie-Technik, 18, (1989), Seiten 14 bis 21. Sie bestehen zum Beispiel aus Kunststofffasern aus Polypropylen oder Polytetrafluorethylen oder aus Glas- oder Metallfasern. Zur Durchströmung des Faserbetts ist ein treibender Differenzdruck von etwa 100 hPa notwendig.

Die erste Reaktionsstufe des neuen Verfahrens führt man als heterogene Reaktion in einem Zweiphasensystem durch, eine Umsetzung, die zum Beispiel in der DE-B-26 27 354 beschrieben ist. Dieser Prozess ist charakterisiert durch das Vorliegen einer organischen Phase, die das olefinische Ausgangsmaterial und das Reaktionsprodukt enthält und einer wässrigen Phase, in der der Katalysator gelöst ist. Als Katalysatoren werden wasserlösliche Rhodium-Komplexverbindungen eingesetzt, die wasserlösliche organische Phosphor(III)-Verbindungen als Liganden enthalten. Beispiele für wasserlösliche Phosphor(III)-Verbindungen, die mit Rhodium Komplexverbindungen bilden, sind Triarylphosphine, Trialkylphosphine und aromatische oder gemischt aliphatisch-aromatische Bisphosphine, deren organische Reste Sulfonsäuregruppen oder Carboxylgruppen enthalten. Ihre Herstellung und Anwendung ist zum Beispiel aus DE-B 26 27 354, EP 0 103 810 B1, EP 0 163 234 B1 und EP 0 571 819 A1 bekannt. Weitere Gruppen geeigneter Verbindungen sind sulfonierte oder carboxylierte organische Bisphosphite sowie heterocyclische Verbindungen des dreibindigen Phosphors (zum Beispiel EP 0 575 785 A1, EP 0 646 588 A1).

Die Bedingungen, unter denen die Umsetzung in der ersten Reaktionsstufe abläuft, können innerhalb weiter Grenzen variiert und den individuellen Gegebenheiten angepasst werden. Sie hängen unter anderem vom Einsatzmaterial, vom ausgewählten Katalysatorsystem und vom angestrebten Umsetzungsgrad ab. Üblicherweise führt man die Hydroformylierung der Einsatzstoffe bei Temperaturen von 50 bis 180°C durch. Bevorzugt hält man Temperaturen von 90 bis 150°C und insbesondere von 110 bis 140°C ein. Der Gesamtdruck erstreckt sich über einen Bereich von 0,4 bis 10 MPa, vorzugsweise 1 bis 6 MPa und insbesondere 1,5 bis 5 MPa. Das molare Verhältnis von Wasserstoff zu Kohlenmonoxid bewegt sich üblicherweise zwischen 1 : 10 bis 10 : 1, Mischungen, die Wasserstoff und Kohlenmonoxid im Verhältnis 3 : 1 bis 1 : 3 und insbesondere etwa 1 : 1, enthalten, sind besonders geeignet.

Die Rhodium-Konzentration beträgt 20 bis 1000 Gew.-ppm, vorzugsweise 50 bis 500 Gew.-ppm und insbesondere 100 bis 300 Gew.-ppm, jeweils bezogen auf die wässrige Katalysatorlösung. Obgleich es möglich ist, als Katalysator die stöchiometrisch zusammengesetzte Rhodium-Phosphor-Komplexverbindung einzusetzen, arbeitet man üblicherweise in Gegenwart von überschüssigem Phosphorliganden, d.h. Ligand, der mit Rhodium keine komplexe Bindung eingegangen ist. Je mol Rhodium wendet man bevorzugt 3 bis 200 mol Phosphor in Form einer wasserlöslichen organischen Phosphorverbindung an. Besonders bewährt haben sich molare Verhältnisse von Rhodium zu Phosphor im Bereich von 1 : 50 bis 1 : 100. Der Rhodium-Phosphor-Komplexkatalysator braucht nicht einheitlich zusammengesetzt sein, sondern kann zum Beispiel aus einem Gemisch von Rhodium-Komplexverbindungen bestehen, die sich durch die Art der Phosphorliganden unterscheiden. Ebenso kann der in der wässrigen Katalysatorlösung enthaltene freie Phosphorligand aus einem Gemisch unterschiedlicher wasserlöslicher organischer Phosphorverbindungen zusammengesetzt sein. Man bildet den Katalysator üblicherweise aus den Komponenten Rhodium oder Rhodiumverbindung, organische Phosphorverbindung und Synthesegas unter den Bedingungen der Hydroformylierungsreaktion im Reaktionsgemisch. Er kann der Reaktionsstufe aber auch präformiert, das heißt gesondert hergestellt, zugeführt werden.

Auch hinsichtlich der verfahrenstechnischen und apparativen Ausgestaltung der ersten Stufe des neuen Verfahrens kann man sich innerhalb weiter Grenzen bewegen. Eine bewährte Ausführungsform der heterogenen Hydroformylierung unter Verwendung einer wässrigen Katalysatorphase ist in der EP 0 103 810 B1 beschrieben. Es hat sich als zweckmäßig erwiesen, die Katalysatorlösung im Kreis zu führen und gegebenenfalls auftretende Katalysatorverluste durch Zufuhr von Frischkatalysator auszugleichen.

Um den Umsatz je Zeiteinheit von olefinisch ungesättigten Verbindungen, die in der wässrigen Katalysatorlösung nur wenig löslich sind, zu erhöhen, kann es sich empfehlen, dieser Lösung ein Phasentransferreagenz (Lösungsvermittler) zuzusetzen. Es verändert die physikalischen Eigenschaften der Grenzflächen zwischen den beiden flüssigen Phasen und erleichtert den Übergang des organischen Reaktanten in die wässrige Katalysatorphase.

Als Lösungsvermittler sind Verbindungen bekannt, deren hydrophile Gruppen ionisch (anionisch oder kationisch) oder nichtionisch sind. Zu den anionenaktiven Verbindungen gehören Natrium-, Kalium- oder Ammoniumsalze von Carbonsäuren, vorzugsweise solchen mit 8 bis 20 Kohlenstoffatomen und insbesondere von gesättigten Fettsäuren mit 12 bis 18 Kohlenstoffatomen, ferner Alkylsulfate, Alkylbenzolsulfonate und Alkylbenzolphosphate. Beispiele für kationische Lösungsvermittler sind Tetraalkylammonium- und N-Alkylpyridiniumsalze. Die nichtionischen Phasentransferreagenzien dissoziieren in wässriger Lösung nicht in Ionen. Zu ihnen zählen Alkylpolyethylenglykole, Alkylphenylpolyethylenglykole, Fettsäurealkylolamine und Trialkylaminoxide. Ferner finden Ampholyte wie Aminocarbonsäuren, Betaine und Sulfobetain als Lösungsvermittler Anwendung. Entsprechende Verfahren sind zum Beispiel in EP 0 157 316 B1 beschrieben.

Schließlich können auch Rhodiumkomplexverbindungen eingesetzt werden, die gleichzeitig Katalysator und Phasentransferreagenz sind. Eine solche Arbeitsweise ist zum Beispiel Gegenstand der EP 0 163 234 B1.

Üblicherweise strebt man in der ersten Reaktionsstufe einen möglichst weitgehenden Umsatz der olefinisch ungesättigten Verbindungen an. In Einzelfällen kann jedoch auch auf einen mehr oder minder großen Teilumsatz hin gearbeitet werden.

Das aus der ersten Reaktionsstufe entweichende Abgas, der Abgasstom, setzt sich zusammen aus dem Abgas, das dem Reaktor unmittelbar entnommen wird, dem Reaktorabgas, um eine Anreicherung von Inerten in dem, im Kreis geführten Gasgemisch zu vermeiden und den gasförmigen Anteilen, die bei der Trennung von Katalysatorlösung und rohem Reaktionsprodukt im Phasentrenner auftreten, dem Produktabgas. Der der ersten Reaktionsstufe entnommene Abgasstrom, also Reaktorabgas und Produktabgas zusammen, besteht im Wesentlichen aus nicht umgesetzter olefinisch ungesättigter Verbindung, Kohlenmonoxid, Kohlendioxid, Wasserstoff und den Hydrierungsprodukten der olefinisch ungesättigten Verbindung und enthält Wasser im Allgemeinen in einer Menge von 1 bis 4 Gew.-%, bezogen auf die gesamte Abgasmenge. Dieses Gasgemisch wird zunächst in einem Abscheider abgekühlt. Im Allgemeinen kühlt man auf eine Temperatur unterhalb von 25°C, vorzugsweise unterhalb von 20°C unter Bildung eines flüssigen Abgaskondensats, das im Wesentlichen aus der eingesetzten olefinisch ungesättigten Verbindung und der entsprechenden, durch Hydrierung gebildeten gesättigten Verbindung besteht und Wasser in einer Menge von 1 bis 4 Gew.-%, bezogen auf die gesamte Menge enthält. Man betreibt den Abscheider bei Reaktordruck oder einem geringfügig geringeren Druck, der sich aus dem Druckverlust zwischen dem ersten Reaktor und dem Abscheider ergibt. Zusätzlich liegt noch eine gasförmige Phase im Abscheider vor, das sogenannte Entspannungsgas. Die abgeschiedene flüssige Phase wird durch einen Koalisierfilter geleitet, der mit einem zusätzlichen Abscheidegefäß verbunden ist. Das sich dabei abscheidende Wasser wird gesammelt und aus dem Prozess ausgeschleust. Durch dieses Zwischenschalten eines Koalisierfilters kann der Wassergehalt im Abgaskondensat auf einen Wert im Bereich von 0,1 bis 1,0 Gew.-% reduziert werden. Der Druckverlust durch das Zwischenschalten eines Koalisierfilters beträgt zwischen 10 und 50 hPa.

Das über den Koalisierfilter geführte flüssige Abgaskondensat und die im Entspannungsschritt im Abscheider anfallende gasförmige Phase, also das Entspannungsgas werden anschließend getrennt oder gemeinsam, gegebenenfalls nach Zumischen von Wasserstoff allein oder im Gemisch mit Kohlenmonoxid, als Einsatzmaterial auf die für die Durchführung der zweiten Hydroformylierungsstufe erforderliche Druckstufe komprimiert und in die zweite Hydroformylierungsstufe eingespeist.

Die zweite Reaktionsstufe wird unabhängig von der ersten betrieben. In ihr werden die im Abgasstrom vorhandenen Restmengen der olefinisch ungesättigten Verbindungen in einem homogenen Reaktionssystem mit Kohlenmonoxid und Wasserstoff umgesetzt. Der Begriff homogenes Reaktionssystem steht für eine im Wesentlichen aus Lösungsmittel, Katalysator, olefinisch ungesättigter Verbindung und Reaktionsprodukt zusammengesetzte homogene Lösung. Als Katalysatoren werden Rhodium-Komplexverbindungen verwendet, die organische Phosphor(III)-Verbindungen als Liganden enthalten. Derartige Komplexverbindungen und ihre Herstellung sind bekannt (zum Beispiel US 3 527 809 A1, US 4 148 830 A1. US 4 247 486 A1, US 4 283 562 A1). Sie können als einheitliche Komplexverbindung oder auch als Gemisch unterschiedlicher Komplexverbindungen eingesetzt werden. Die Rhodium-Konzentration im Reaktionsmedium erstreckt sich über einen Bereich von etwa 1 bis etwa 1000 Gew.-ppm und beträgt vorzugsweise 10 bis 500 Gew.-ppm. Insbesondere wendet man Rhodium in Konzentrationen von 10 bis 200 Gew.-ppm, jeweils bezogen auf das homogene Reaktionsgemisch, an. Wie in der ersten Stufe kann als Katalysator die stöchiometrisch zusammengesetzte Rhodium-Komplexverbindung Anwendung finden. Es hat sich jedoch als zweckmäßig erwiesen, die Hydroformylierung in Gegenwart eines Katalysatorsystems aus Rhodium-Phosphor-Komplexverbindung und freiem, d.h. überschüssigem Phosphorliganden durchzuführen, der mit Rhodium keine Komplexverbindung mehr eingeht. Der freie Phosphorligand kann der gleiche sein, wie in der Rhodium-Komplexverbindung, es können aber auch von diesem verschiedene Liganden eingesetzt werden. Der freie Ligand kann eine einheitliche Verbindung sein oder aus einem Gemisch verschiedener Organophosphorverbindungen bestehen. Beispiele für Rhodium-Phosphor-Komplexverbindungen, die als Katalysatoren Anwendung finden können, sind in US 3 527 809 A1 beschrieben. Zu den bevorzugten Liganden in den Rhodium-Komplexkatalysatoren zählen z.B. Triarylphosphine wie Triphenylphosphin, Trialkylphosphine wie Tri(n-octyl)phosphin, Trilaurylphosphin, Tri(cyclohexyl)phosphin, Alkylphenylphosphine, Cycloalkylphenylphosphine und organische Bisphosphite. Wegen seiner leichten Zugänglichkeit wird Triphenylphosphin besonders häufig angewandt.

Üblicherweise beträgt das molare Verhältnis von Rhodium zu Phosphor 1 : 1 bis 1 : 300, jedoch kann der molare Anteil des Phosphors in Form organischer Phosphorverbindungen auch höher sein. Vorzugsweise setzt man Rhodium und organisch gebundenen Phosphor in molaren Verhältnissen von 1 : 3 bis 1 : 200 ein. Bei Anwendung von Triarylphosphinen haben sich insbesondere Rh/P-Molverhältnisse von 1 : 30 bis 1 : 150 bewährt.

Die Hydroformylierungsreaktion wird in Gegenwart eines Lösungsmittels ausgeführt. Als Lösungsmittel werden organische Verbindungen eingesetzt, in denen Ausgangsmaterial, Reaktionsprodukt und Katalysatorsystem löslich sind. Beispiele für solche Verbindungen sind aromatische Kohlenwasserstoffe wie Benzol, Toluol oder die Xylole. Andere gebräuchliche Lösungsmittel sind Paraffinöl, Ketone oder Ether. Als besonders geeignete Lösungsmittel haben sich die höhersiedenden Kondensationsverbindungen der Aldehyde erwiesen, die als Nebenprodukte bei der Hydroformylierung entstehen. Der Anteil des Lösungsmittels im Reaktionsmedium kann über einen weiten Konzentrationsbereich variiert werden und beträgt üblicherweise zwischen 20 und 90 Gew.-%, vorzugsweise 50 bis 80 Gew.-%, bezogen auf das Reaktionsgemisch.

Der Reaktionsdruck in der zweiten Stufe des Gesamtprozesses liegt im Bereich von 15 bis 40 MPa. Besonders bewährt hat es sich, Drücke zwischen 15 und 35 MPa, vorzugsweise 20 bis 30 MPa einzuhalten. Derartige Bereiche sind für Hydroformylierungen in homogenen Reaktionssystemen und in Gegenwart von Komplexverbindungen des Rhodiums mit organischen Phosphorverbindungen ungewöhnlich, unabhängig davon, ob die Umsetzung ein- oder mehrstufig erfolgt. Das Volumenverhältnis von Wasserstoff zu Kohlenmonoxid beträgt 1 : 10 bis 10 : 1, vorzugsweise 1 : 3 bis 3 : 1 und insbesondere 1 : 1.

Die Reaktionstemperaturen betragen in der zweiten Stufe des neuen Prozesses 50 bis 160°C. Temperaturen von 60 bis 150°C und insbesondere 75 bis 140°C werden bevorzugt.

Wie bereits erwähnt, wird das Reaktionsprodukt der ersten Reaktionsstufe in einem Phasentrenner von der wässrigen Katalysatorlösung, die in den Prozess zurückgeführt wird, getrennt. Nach einer bewährten Ausführungsform führt man den Rohaldehyd in einer Stripp-Kolonne im Gegenstrom zu frischem Synthesegas. Hierbei wird Wärme vom Aldehyd auf das Synthesegas übertragen und die im Aldehyd gelöste olefinische Verbindung aus dem Rohprodukt ausgetrieben und zusammen mit dem erwärmten Synthesegas erneut der Reaktion zugeleitet. Das Umsetzungsprodukt der zweiten Reaktionsstufe wird vom Katalysator abdestilliert. Es kann mit dem Produkt der ersten Stufe vereinigt und weiter verarbeitet, zum Beispiel destilliert, werden. Aufgrund der erfindungsgemäß vorgenommenen mechanischen Wasserabtrennung kann man bei den Destillationsstufen unter sehr schonenden Bedingungen und unter geringerem Energieeinsatz arbeiten, um zu wasserfreien Produkten zu gelangen.

Der nach Abtrennung des Aldehyds verbleibende, Katalysator enthaltende Destillationsrückstand der zweiten Reaktionsstufe, wird gegebenenfalls nach Zusatz von Frischkatalysator und Entnahme eines Teils der im Verlauf der Reaktion gebildeten Aldehydkondensationsprodukte in die Reaktionszone rezirkuliert.

Das erfindungsgemäße Verfahren kann auf olefinisch ungesättigte Verbindungen beliebiger Struktur angewandt werden. Dementsprechend sind als Ausgangsmaterial geeignet sowohl Olefine mit innenständiger als auch mit endständiger Doppelbindung und ebenso geradkettige oder verzweigte Olefine. Überdies können die Olefine auch noch durch funktionelle Gruppen substituiert sein, insbesondere solche, die im Verlauf der Reaktion nicht verändert werden. Auch mehrfach olefinisch ungesättigte Verbindungen kommen als Einsatzstoffe in Betracht. Besonders bewährt hat sich das Verfahren bei der Hydroformylierung olefinisch ungesättigter Kohlenwasserstoffe mit 3 bis 6 Kohlenstoffatomen im Molekül, vorzugsweise Propylen und die isomeren Butene.

Die Erfindung wird in den nachfolgenden Beispielen näher erläutert, jedoch nicht auf die beschriebenen Ausführungsformen beschränkt.

### Beispiel 1

### Erste Reaktionsstufe

In einem Reaktor wurde unter intensivem Rühren bei einem Synthesegasdruck (CO : H₂ = 1 : 1) von 5 MPa und bei einer Temperatur von 122°C aus Rhodiumacetat und Triphenylphosphintrisulfonat-Na (TPPTS) der aktive, wasserlösliche Katalysator HRhCO(TPPTS)₃ hergestellt. Rhodiumverbindung und TPPTS wurden in einer solchen Menge eingesetzt, dass die Rhodiumkonzentration in der wässrigen Katalysatorlösung 300 Gew.-ppm und das Molverhältnis von Rhodium zu Phosphor etwa 1 : 100 betrug.

Über einen Verteilerring am Boden des Reaktors leitete man vorgewärmtes Propylen in die Reaktionszone und setzte dort das Olefin mit Kohlenmonoxid und Wasserstoff bei 122°C und 5 MPa um. Der aus gasförmigen und flüssigen Bestandteilen bestehende Produktstrom wurde dem oberen Teil des Reaktors entnommen und einem Phasentrenner zugeführt, in dem die Trennung der wässrigen Katalysatorlösung vom rohen organischen Reaktionsprodukt und vom Produktabgas erfolgte. Das Produktabgas wurde mit dem Reaktorabgas zum Abgasstrom vereinigt. Reaktorabgas wurde dem Reaktor entnommen, um eine Anreicherung von Inerten im Gasgemisch zu vermeiden, das im Kreis geführt wurde. Der abgeführte Abgasstrom wurde anschließend auf eine Temperatur unterhalb 25°C gekühlt, wobei sich ein flüssiges Kondensat, das Abgaskondensat, abscheidete, das im Wesentlichen aus Propylen und Propan besteht. Der Wassergehalt im Abgaskondensat betrug etwa 2 Gew.-%.

### Zweite Reaktionsstufe

Das Abgaskondensat aus der ersten Reaktionsstufe wurde über eine Koalisierfilter mit einer Belastung von 0,5 V/V·h geleitet, wobei ein Druckverlust von 20 hPa beobachtet wurde. Das sich dabei abscheidende Wasser wurde in einem Gefäß aufgefangen. Nach Austritt durch das Filter wies das flüssige Abgaskondensat einen Wassergehalt von noch 0,5 Gew.-% auf.

Das so behandelte flüssige Abgaskondensat und die bei der Abscheidung des flüssigen Abgaskondensats angefallene gasförmige Phase, das Entspannungsgas, wurde zu einem Einsatzgemisch mit folgender Zusammensetzung (alle Angaben in Gew.-%) vereinigt.

| | |
|---|---|
| Wasserstoff | 2,20 |
| Kohlenmonoxid | 29,94 |
| Kohlendioxid | 0,31 |
| Inerte | 1,29 |
| Propylen | 40,01 |
| Propan | 10,19 |
| n-Butyraldehyd | 7,60 |
| Isobutyraldehyd | 0,61 |
| Butanole | 1,27 |
| C8-Komponenten | 1,60 |
| > C8-Komponenten | 2,76 |
| Triphenylphosphin | 1,21 |
| Triphenylphosphinoxid | 0,50 |
| Wasser | 0,50 |
| Schwefel | Spuren |

Dieses Einsatzgemisch wurde gegebenenfalls durch Wasserstoff und/oder Kohlenmonoxid und/oder Propylen ergänzt und zusammen mit der Katalysatorlösung auf 21 MPa komprimiert und dem zweiten Reaktor zugeführt. Der Katalysator bestand aus höhersiedenden Aldehydkondensationsprodukten, in denen Triphenylphosphin (TPP) und die Rhodium-Komplexverbindung HRhCO(TPP)₃ gelöst sind. Das Einsatzgemisch wurde vom Boden her in den Reaktor eingeführt.

Das Molverhältnis von Phosphor zu Rhodium betrug etwa 80 : 1. Die Umsetzung der Reaktanten erfolgte bei 140°C. 99 % des eingesetzten Propylens (d.h. des nicht umgesetzten Propylens der ersten Reaktionsstufe und gegebenenfalls dem Gemisch aus Abgaskondensat und Entspannungsgas vor Eintritt in den zweiten Reaktor zugesetztes Propylen) wurden in Aldehyd umgewandelt. Der den zweiten Reaktor verlassende Produktstrom wurde über einen Abscheider entspannt. Neben einer flüssigen Phase, dem Aldehyd-Rohprodukt, erhielt man eine gasförmige Phase, ebenfalls ein Entspannungsgas, das man zur Abscheidung restlichen Aldehyds fraktioniert kondensierte.

Der Rohaldehyd wurde in einer ersten Kolonne vom Katalysator abdestilliert und in einer zweiten Kolonne in n- und i-Butyraldehyd getrennt. Der Katalysator, er fiel als flüssiger Rückstand in der ersten Kolonne an, wurde zum überwiegenden Teil in den Reaktor zurückgeführt. Lediglich ein kleiner Teilstrom wurde in einer solchen Menge abgetrennt, dass die Konzentration der als Lösungsmittel für den Rhodiumkatalysator verwendeten höhersiedenden Aldehydkondensationsprodukte im zweiten Reaktor etwa konstant blieb.

## Patentansprüche

1. Verfahren zur Hydroformylierung olefinisch ungesättigter Verbindungen, wobei die Reaktion der ersten Reaktionsstufe in einem heterogen Reaktionssystem unter Verwendung einer wässrigen Lösung, wasserlösliche organische Phosphor(III)-Verbindungen in komplexer Bindung enthaltender Rhodiumverbindungen als Katalysatoren bei Drücken von 0,4 bis 10 MPa erfolgt und Abgas gebildet wird, und wobei das Abgas der ersten Reaktionsstufe einer zweiten Reaktionsstufe zugeführt wird, in der im Abgas noch vorhandene Restmengen der olefinisch ungesättigten Verbindungen in einem homogenen Reaktionssystem in Gegenwart von Komplexverbindungen des Rhodiums mit organischen Phosphor(III)-Verbindungen als Katalysatoren bei Drücken von 15 bis 40 MPa umgesetzt werden, **dadurch gekennzeichnet, dass** man das Abgas der ersten Reaktionsstufe kühlt und ein Abgaskondensat bildet, das man durch einen Koalisierfilter leitet, um Wasser und darin enthaltene Inhaltsstoffe zu entfernen, und anschlieβend der zweiten Reaktionsstufe zuführt.

2. Verfahren gemäß Anspruch 1, **dadurch gekennzeichnet, dass** man das Abgas der ersten Reaktionsstufe auf eine Temperatur unterhalb von 25°C, kühlt.

3. Verfahren gemäß Anspruch 2, **dadurch gekennzeichnet, dass** man das Abgas der ersten Reaktionsstufe auf eine Temperatur unterhalb von 20°C kühlt.

4. Verfahren gemäß einem oder mehreren der Ansprüche 1 bis 3, **dadurch gekennzeichnet, dass** man die bei der Bildung des Abgaskondensats anfallende gasförmige Phase gemeinsam mit dem durch das Koalisierfilter geleiteten Abgaskondensat, gegebenenfalls nach Zumischen von Wasserstoff allein oder im Gemisch mit Kohlenmonoxid, der zweiten Reaktionsstufe zuführt.

5. Verfahren nach einem oder mehreren der Ansprüche 1 bis 4, **dadurch gekennzeichnet, dass** die Hydroformylierung in der ersten Reaktionsstufe bei Temperaturen von 50 bis 180°C und einer Rhodiumkonzentration von 20 bis 1000 Gew.-ppm, bezogen auf die wässrige Katalysatorlösung erfolgt und das molare Verhältnis von Rhodium zu Phosphor in der Katalysatorlösung 1 : 3 bis 1 : 200 beträgt.

6. Verfahren nach einem oder mehreren der Ansprüche 1 bis 5, **dadurch gekennzeichnet, dass** der Druck in der ersten Reaktionsstufe 1 bis 6 MPa beträgt.

7. Verfahren nach einem oder mehreren der Ansprüche 1 bis 6, **dadurch gekennzeichnet, dass** die Temperatur in der ersten Reaktionsstufe 90 bis 150°C beträgt.

8. Verfahren nach einem oder mehreren der Ansprüche 1 bis 7, **dadurch gekennzeichnet, dass** die Konzentration des Rhodiums in der wässrigen Katalysatorlösung 50 bis 500 Gew.-ppm beträgt.

9. Verfahren nach einem oder mehreren der Ansprüche 1 bis 8 **dadurch gekennzeichnet, dass** das molare Verhältnis von Rhodium zu Phosphor in der wässrigen Katalysatorlösung 1 : 50 bis 1 : 100 beträgt.

10. Verfahren nach einem oder mehreren der Ansprüche 1 bis 9, **dadurch gekennzeichnet, dass** als wasserlösliche organische Phosphor(III)-Verbindungen sulfonierte oder carboxylierte Triarylphosphine, Trialkylphosphine, aromatische oder gemischt aliphatisch-aromatische Bisphosphine oder Bisphosphite eingesetzt werden.

11. Verfahren nach einem oder mehreren der Ansprüche 1 bis 10, **dadurch gekennzeichnet, dass** die Hydroformylierung in der zweiten Reaktionsstufe in Gegenwart eines Lösungsmittels bei Temperaturen von 50 bis 160°C und einer Rhodiumkonzentration von 1 bis 1000 Gew.-ppm, bezogen auf das homogene Reaktionsgemisch, erfolgt und das molare Verhältnis von Rhodium zu Phosphor im Reaktionsgemisch 1 : 1 bis 1 : 300 beträgt.

12. Verfahren nach Anspruch 11, **dadurch gekennzeichnet, dass** der Druck in der zweiten Reaktionsstufe 15 bis 35 MPa beträgt.

13. Verfahren nach Anspruch 11 oder 12, **dadurch gekennzeichnet, dass** die Temperatur in der zweiten Reaktionsstufe 60 bis 150°C beträgt.

14. Verfahren nach einem oder mehreren der Ansprüche 11 bis 13, **dadurch gekennzeichnet, dass** die Rhodiumkonzentration in der zweiten Reaktionsstufe 10 bis 500 Gew.-ppm, bezogen auf das Reaktionsgemisch, beträgt..

15. Verfahren nach einem oder mehreren der Ansprüche 11 bis 14, **dadurch gekennzeichnet, dass** in der zweiten Reaktionsstufe das molare Verhältnis von Rhodium zu Phosphor im Reaktionsgemisch 1 : 3 bis 1 : 200 beträgt.

16. Verfahren nach einem oder mehreren der Ansprüche 11 bis 15, **dadurch gekennzeichnet, dass** als organische Phosphor(III)-Verbindungen aliphatische, aromatische oder gemischt aliphatisch-aromatische Phosphine oder Bisphosphite eingesetzt werden.

17. Verfahren nach Anspruch 16, **dadurch gekennzeichnet, dass** die organische Phosphor(III)-verbindung ein Triarylphosphin ist und das molare Verhältnis von Rhodium zu Phosphor im Reaktionsgemisch 1 : 30 bis 1 : 150 beträgt.

18. Verfahren nach Anspruch 17, **dadurch gekennzeichnet, dass** das Triarylphosphin Triphenylphosphin ist.

## Claims

1. A process for the hydroformylation of olefinically unsaturated compounds, in which the reaction of the first reaction stage is carried out in a heterogeneous reaction system using an aqueous solution comprising rhodium compounds comprising complexed watersoluble organic phosphorus(III) compounds as catalysts at pressures of from 0.4 to 10 MPa and offgas is formed, and the offgas from the first reaction stage is fed to a second reaction stage in which residual amounts of olefinically unsaturated compounds still present in the offgas are reacted in a homogeneous reaction system in the presence of complexes of rhodium with organic phosphorus(III) compounds as catalysts at pressures of from 15 to 40 MPa, wherein the offgas from the first reaction stage is cooled to form an offgas condensate which is passed through a coalescing filter in order to remove water and constituents present therein and subsequently fed to the second reaction stage.

2. The process as claimed in claim 1, wherein the offgas from the first reaction stage is cooled to a temperature below 25°C.

3. The process as claimed in claim 2, wherein the offgas from the first reaction stage is cooled to a temperature below 20°C.

4. The process as claimed in one or more of claims 1 to 3, wherein the gaseous phase obtained in the formation of the offgas condensate is fed together with the offgas condensate passed through the coalescing filter, if appropriate after addition of hydrogen either alone or in admixture with carbon monoxide, to the second reaction stage.

5. The process as claimed in one or more of claims 1 to 4, wherein the hydroformylation in the first reaction stage is carried out at temperatures of from 50 to 180°C and a rhodium concentration of from 20 to 1000 ppm by weight, based on the aqueous catalyst solution, and the molar ratio of rhodium to phosphorus in the catalyst solution is from 1:3 to 1:200.

6. The process as claimed in one or more of claims 1 to 5, wherein the pressure in the first reaction stage is from 1 to 6 MPa.

7. The process as claimed in one or more of claims 1 to 6, wherein the temperature in the first reaction stage is from 90 to 150°C.

8. The process as claimed in one or more of claims 1 to 7, wherein the concentration of rhodium in the aqueous catalyst solution is from 50 to 500 ppm by weight.

9. The process as claimed in one or more of claims 1 to 8, wherein the molar ratio of rhodium to phosphorus in the aqueous catalyst solution is from 1:50 to 1:100.

10. The process as claimed in one or more of claims 1 to 9, wherein sulfonated or carboxylated triarylphosphines, trialkylphosphines, aromatic or mixed aliphatic-aromatic bisphosphines or bisphosphites are used as water-soluble organic phosphorus(III) compounds.

11. The process as claimed in one or more of claims 1 to 10, wherein the hydroformylation in the second reaction stage is carried out in the presence of a solvent at temperatures of from 50 to 160°C and a rhodium concentration of from 1 to 1000 ppm by weight, based on the homogeneous reaction mixture, and the molar ratio of rhodium to phosphorus in the reaction mixture is from 1:1 to 1:300.

12. The process as claimed in claim 11, wherein the pressure in the second reaction stage is from 15 to 35 MPa.

13. The process as claimed in claim 11 or 12, wherein the temperature in the second reaction stage is from 60 to 150°C.

14. The process as claimed in one or more of claims 11 to 13, wherein the rhodium concentration in the second reaction stage is from 10 to 500 ppm by weight, based on the reaction mixture.

15. The process as claimed in one or more of claims 11 to 14, wherein the molar ratio of rhodium to phosphorus in the reaction mixture in the second reaction stage is from 1:3 to 1:200.

16. The process as claimed in one or more of claims 11 to 15, wherein aliphatic, aromatic or mixed aliphatic-aromatic phosphines or bisphosphites are used as organic phosphorus(III) compounds.

17. The process as claimed in claim 16, wherein the organic phosphorus(III) compound is a triarylphosphine and the molar ratio of rhodium to phosphorus in the reaction mixture is from 1:30 to 1:150.

18. The process as claimed in claim 17, wherein the triarylphosphine is triphenylphosphine.

## Revendications

1. Procédé d'hydroformylation de composés oléfiniquement insaturés, la réaction de la première étape de réaction ayant lieu dans un système réactionnel hétérogène en utilisant une solution aqueuse de composés de rhodium contenant en liaison complexe des composés de phosphore (III) organiques solubles dans l'eau en tant que catalyseurs à des pressions de 0,4 à 10 MPa et un gaz d'échappement étant formé, et le gaz d'échappement de la première étape de réaction étant introduit dans une seconde étape de réaction, lors de laquelle les quantités résiduelles des composés oléfiniquement insaturés encore présentes dans le gaz d'échappement sont transformées dans un système réactionnel homogène en présence de composés complexes de rhodium avec des composés de phosphore (III) organiques en tant que catalyseurs à des pressions de 15 à 40 MPa, **caractérisé en ce que** le gaz d'échappement de la première étape de réaction est refroidi et forme un condensat de gaz d'échappement, qui est passé au travers d'un filtre à coalescence pour éliminer l'eau et les composants contenus dans celle-ci, puis introduit dans la seconde étape de réaction.

2. Procédé selon la revendication 1, **caractérisé en ce que** le gaz d'échappement de la première étape de réaction est refroidi à une température inférieure à 25 °C.

3. Procédé selon la revendication 2, **caractérisé en ce que** le gaz d'échappement de la première étape de réaction est refroidi à une température inférieure à 20 °C.

4. Procédé selon une ou plusieurs des revendications 1 à 3, **caractérisé en ce que** la phase gazeuse formée lors de la formation du condensat de gaz d'échappement est introduite dans la seconde étape de réaction conjointement avec le condensat de gaz d'échappement ayant traversé le filtre à coalescence, éventuellement après incorporation d'hydrogène seul ou en mélange avec du monoxyde de carbone.

5. Procédé selon une ou plusieurs des revendications 1 à 4, **caractérisé en ce que** l'hydroformylation lors de la première étape de réaction a lieu à des températures de 50 à 180 °C et à une concentration de rhodium de 20 à 1 000 ppm en poids, par rapport à la solution aqueuse de catalyseur, et le rapport molaire entre le rhodium et le phosphore dans la solution de catalyseur est de 1:3 à 1:200.

6. Procédé selon une ou plusieurs des revendications 1 à 5, **caractérisé en ce que** la pression lors de la première étape de réaction est de 1 à 6 MPa.

7. Procédé selon une ou plusieurs des revendications 1 à 6, **caractérisé en ce que** la température lors de la première étape de réaction est de 90 à 150 °C.

8. Procédé selon une ou plusieurs des revendications 1 à 7, **caractérisé en ce que** la concentration de rhodium dans la solution aqueuse de catalyseur est de 50 à 500 ppm en poids.

9. Procédé selon une ou plusieurs des revendications 1 à 8, **caractérisé en ce que** le rapport molaire entre le rhodium et le phosphore dans la solution aqueuse de catalyseur est de 1:50 à 1:100.

10. Procédé selon une ou plusieurs des revendications 1 à 9, **caractérisé en ce que** des bisphosphites ou des bisphosphines aromatiques ou aliphatiques-aromatiques mixtes, des trialkylphosphines, des triarylphosphines sulfonés ou carboxylés sont utilisés en tant que composés de phosphore (III) organiques solubles dans l'eau.

11. Procédé selon une ou plusieurs des revendications 1 à 10, **caractérisé en ce que** l'hydroformylation lors de la seconde étape de réaction a lieu en présence d'un solvant à des températures de 50 à 160 °C et à une concentration de rhodium de 1 à 1 000 ppm en poids, par rapport au mélange réactionnel homogène, et le rapport molaire entre le rhodium et le phosphore dans le mélange réactionnel est de 1:1 à 1:300.

12. Procédé selon la revendication 11, **caractérisé en ce que** la pression lors de la seconde étape de réaction est de 15 à 35 MPa.

13. Procédé selon la revendication 11 ou 12, **caractérisé en ce que** la température lors de la seconde étape de réaction est de 60 à 150 °C.

14. Procédé selon une ou plusieurs des revendications 11 à 13, **caractérisé en ce que** la concentration de rhodium lors de la seconde étape de réaction est de 10 à 500 ppm en poids, par rapport au mélange réactionnel.

15. Procédé selon une ou plusieurs des revendications 11 à 14, **caractérisé en ce que** le rapport molaire entre le rhodium et le phosphore dans le mélange réactionnel lors de la seconde étape de réaction est de 1:3 à 1:200.

16. Procédé selon une ou plusieurs des revendications 11 à 15, **caractérisé en ce que** des biphosphites ou des phosphines aliphatiques, aromatiques ou aliphatiques-aromatiques mixtes sont utilisés en tant que composés de phosphore (III) organiques.

17. Procédé selon la revendication 16, **caractérisé en ce que** le composé de phosphore (III) organique est une triarylphosphine et le rapport molaire entre le rhodium et le phosphore dans le mélange réactionnel est de 1:30 à 1:150.

18. Procédé selon la revendication 17, **caractérisé en ce que** la triarylphosphine est la triphénylphosphine.
